# EUROPEAN PATENT APPLICATION

(11) **EP 4 613 851 A1**
(43) Date of publication of application: **10.09.2025**
(21) Application number: 23885763.5
(22) Date of filing: 31.10.2023
(51) Int. Cl.: C12N 5/0775, A61K 35/28, A61P 15/08, C12N 5/076

(54) **SPERM TREATMENT COMPOSITION, SPERM MOTILITY IMPROVING AGENT, SPERM FERTILITY MAINTAINING AGENT, SPERM MOTILITY IMPROVING METHOD, AND SPERM FERTILITY MAINTAINING METHOD**

(30) Priority: 01.11.2022 JP 2022175838
(71) Applicant: Hiroshima University, Higashihiroshima-shi Hiroshima 739-8511 (JP); Rohto Pharmaceutical Co., Ltd., Osaka-shi, Osaka 544-8666 (JP)
(72) Inventor: SHIMADA, Masayuki, Higashi-Hiroshima City, Hiroshima 7398511 (JP); UMEHARA, Takashi, Higashi-Hiroshima City, Hiroshima 7398511 (JP); ISHIKAWA, Noriyasu, Seika-cho Soraku-gun, Kyoto 619-0237 (JP); TAKIJIRI, Takashi, Osaka-shi, Osaka 544-8666 (JP)
(74) Representative: Mewburn Ellis LLP
(86) International application number: PCT/JP2023/039262
(87) International publication number: WO 2024/096009

(57) **Abstract**

The purpose of the present invention is to provide sperm having high motility. The present invention is a sperm treatment composition that contains an exosome. A sperm treatment composition according to the present invention is used as a sperm preparation solution, a sperm diluent, a sperm storage solution, an artificial insemination solution, an in vitro fertilization solution, a sperm motility improving solution, or a sperm fertility maintaining solution.

## Description

### TECHNICAL FIELD

The present invention relates to a composition for treating sperm, an agent for improving sperm motility, an agent for retaining sperm fertility, a method for improving sperm motility, and a method for retaining sperm fertility.

### BACKGROUND ART

"Infertility" refers to the inability to conceive for a certain period despite unprotected sexual intercourse between a healthy man and woman who wish to conceive, and this "certain period " is defined as "generally one year" by the Japan Society of Obstetrics and Gynecology. It is estimated that about one in ten couples are infertile, but in recent years, the age at which people think about conception has been rising, and it is known that both men and women become less likely to become pregnant as they age, and thus it is said that infertility rate is even higher.

Infertility may be caused by the male, by the female, or both, or by unknown cause, but it is said that about half of all cases the cause is also on the male side. Examples of a known cause of such male infertility include a sexual dysfunction such as spermatogenesis dysfunction, obstruction of sperm transport, erectile dysfunction (ED), or intravaginal ejaculation dysfunction, and aging. In men, it is said that the quality of sperm gradually deteriorates from around the age of 35. As people have been getting married later in life in recent years, it is said that about 10% of men have a problem with their semen findings even before marriage.

Infertility treatment is carried out by selecting the optimal treatment method according to the cause. Major treatment methods include a timing method, an ovulation induction method, artificial insemination, and even an assisted reproductive technique such as in vitro fertilization. Artificial insemination (AIH) is a treatment method involving injecting sperm into the uterine cavity in order to deliver sufficient sperm necessary for fertilization to the ampulla of the fallopian tube, which is the site of fertilization. AIH is indicated for oligozoospermia (sperm concentration of 15 million/ml or less), asthenospermia (motility rate of 40% or less), sexual intercourse dysfunction, sperm cervical mucus incompatibility (failing the Huhner test), a case of anti-sperm antibody retention, and a case of unexplained infertility. However, it is said that the limit of AIH is a total motile sperm count after treating of 1 million to 5 million, and there are also many cases in which it is difficult to satisfy this limit. In addition, when frozen semen is used for artificial insemination, the motility of the sperm decreases because of freezing, and the conception rate cannot currently be said to be high. Therefore, development of a method for enhancing the motility of sperm is desired.

Mesenchymal stem cells are multipotent progenitor cells that were first isolated from bone marrow by Friedenstein (1982) (see Non Patent Document 1). It has been revealed that mesenchymal stem cells are present in various tissues such as bone marrow, umbilical cord, and fat, and mesenchymal stem cell transplantation is expected as a new therapeutic method for various intractable diseases (see Patent Documents 1 and 2). Recently, it has been known that cells having comparable functions are present in stromal cells in adipose tissue, placenta, umbilical cord, egg membrane, and the like. Therefore, mesenchymal stem cells are sometimes called mesenchymal stromal cells.

### PRIOR ART DOCUMENTS

### Patent Document

Patent Document 1: JP 2012-157263 A
Patent Document 2: JP 2012-508733 A

### Non Patent Document

Non Patent Document 1: Pittenger F. M. et al., Science 284, pp.143-147, 1999

### SUMMARY OF INVENTION

### Technical Problem

Under the circumstances described above, an object of the present invention is to provide sperm having excellent motility.

### Solution to Problem

The present inventors have carried out intensive research in order to solve the above problem and as a result, found that exosomes increase the motion performance of sperm, and completed the present invention. According to the present invention, sperm having high motility and suppressed sperm acrosome reaction can be provided. That is, the gist of the present invention is as follows.
[1] A composition for treating sperm, comprising exosomes.
[2] The composition for treating sperm according to [1], wherein the composition is a sperm conditioning solution, a sperm dilution solution, a sperm preservation solution, an artificial insemination solution, an in vitro fertilization solution, a solution for improving sperm motility, or a solution for retaining sperm fertility.
[3] The composition for treating sperm according to [1], wherein the exosomes are derived from a mesenchymal stem cell culture supernatant.
[4] The composition for treating sperm according to [1], wherein the exosomes are isolated exosomes.
[5] An agent for improving sperm motility, comprising exosomes.
[6] An agent for retaining sperm fertility, comprising exosomes.
[7] A method for improving sperm motility, comprising culturing sperm in a solution comprising exosomes.
[8] A method for retaining sperm fertility, comprising culturing sperm in a solution comprising exosomes.

### Advantageous Effects of Invention

According to the present invention, it is possible to provide sperm with high motility and suppressed acrosome reaction. The sperm prepared by the present invention has higher motility and a suppressed acrosome reaction compared to sperm prepared by conventional methods, resulting in a significantly higher success rate of fertilization.

### BRIEF DESCRIPTION OF DRAWINGS

[Fig. 1] Fig. 1 is a diagram showing the motility (straight-line velocity) of mouse sperm under each condition.
[Fig. 2] Fig. 2 is a diagram showing the motility of mouse sperm under each condition.
[Fig. 3] Fig. 3 is a diagram showing the motility (linearity) of mouse sperm under each condition.
[Fig. 4] Fig. 4 is a diagram showing the proportion of acrosome marker positive sperm under each condition for mouse sperm.
[Fig. 5] Fig. 5 is a diagram showing the proportion of acrosome marker positive sperm under each condition for cattle sperm.
[Fig. 6] Fig. 6 is a diagram showing the change in oxygen consumption rate of mouse sperm over time under each condition.
[Fig. 7] Fig. 7 is a diagram showing a fluorescence staining image of mouse sperm treated with fluorescence-labeled exosomes.
[Fig. 8] Fig. 8 is a diagram showing a fluorescence staining image of mouse sperm treated with fluorescence-labeled exosomes (after quenching).

### DESCRIPTION OF EMBODIMENTS

Hereinafter, the composition for treating sperm, the agent for improving sperm motility, the agent for retaining sperm fertility, the method for improving sperm motility, and the method for retaining sperm fertility according to the present invention will be described in detail.

### [Composition for treating sperm]

The composition for treating sperm according to the present invention is characterized by including exosomes. The composition for treating sperm according to the present invention can provide sperm having high motility and suppressed sperm acrosome reaction by including exosomes. Sperm treated with the composition for treating sperm according to the present invention and thereby having improved or increased motility and suppressed acrosome reaction can be expected to, for example, increase fertilization efficiency or conception rate in artificial insemination or in vitro fertilization, or promote early development.

The motility of sperm can be expressed by a sperm motion parameter. Examples of such a motion parameter include the amplitude of the lateral oscillatory movement of the sperm head (amplitude of lateral head displacement; ALH), the number of lateral oscillatory movements of the sperm head (beat cross frequency; BCF), the velocity based on the total distance traveled by sperm (curvilinear velocity; VCL), and the velocity based on the straight-line distance traveled by sperm (straight-line velocity; VSL). ALH/VCL (value obtained by dividing ALH by VCL) indicates the degree of motility in zigzag movement characteristic of sperm. In addition, BCF/VSL (value obtained by dividing BCF by VSL) indicates the degree of motility in straight-line movement of sperm.

### <Exosomes>

The exosomes in the present invention are endoplasmic reticula derived from endosomes of cells, and vesicles that are released from cells and have a size observable with an electron microscope. For the specific size of the exosomes, the average particle size is 1 nm to 1,000 nm, preferably 10 nm to 500 nm, and more preferably 30 nm to 200 nm. The average particle size is the average value of the diameters of particles in measurement by means of dynamic light scattering or an electron microscope. Each of the exosomes can have a lipid bilayer surrounding a biomolecule. The exosomes may include, for example, membrane particles, membrane vesicles, microvesicles, nanovesicles, micro-endoplasmic reticula (microvesicles, average particle size: 30 to 1,000 nm), exosome-like vesicles, ectosome-like vesicles, ectosomes, and/or what are called exovesicles. Exosomes derived from different types of cells can be distinguished by intracellular origin, exosome density in sucrose, shape, precipitation velocity, lipid composition, protein markers, and manner of secretion (that is, after signaling (inductive) or spontaneous (constitutive)). In density gradient centrifugation, for example, exosomes are fractionated into 1.0 to 1.5 g/mL, preferably into 1.1 to 1.3 g/mL. Furthermore, exosomes contain any of phosphatidylserine, cholesterol, sphingomyelin, and ceramide as a constituent lipid.

In the exosomes in the present invention, various proteins derived from secretory cells, including protein derived from endosomes, protein involved in intracellular transport, and protein derived from cell membranes as well as RNA are contained, and a cell membrane of a secretory cell and lipid derived from endosome membranes are also contained. Examples of protein derived from endosomes include ESCRTs and TSG101, examples of protein involved in intracellular transport include Rab and GTPase, examples of protein derived from cell membranes include CD9, CD63, and CD81, and examples of lipid derived from endosome membranes include cholesterol and sphingomyelin.

Examples of other protein and fatty acid contained in the exosomes in the present invention include IL-10, HGF (Hepatocyte Growth Factor), pelargonic acid, lauric acid, myristic acid, pentadecanoic acid, isopentadecanoic acid, palmitic acid, isopalmitic acid, margaric acid, isoheptadecanoic acid, stearic acid, isostearic acid, oleic acid, elaidic acid, linoleic acid, nonadecylic acid, isononadecylic acid, arachidic acid, 11Z-eicosenoic acid, dihomo-γ-linolenic acid, arachidonic acid, erucic acid, 13Z,16Z-docosadienoic acid, 13Z,16Z,19Z-docosadienoic acid, adrenic acid, and clupanodonic acid, preferably IL-10, HGF, pelargonic acid, lauric acid, myristic acid, pentadecanoic acid, isopentadecanoic acid, palmitic acid, isopalmitic acid, margaric acid, isoheptadecanoic acid, stearic acid, isostearic acid, oleic acid, elaidic acid, linoleic acid, arachidic acid, 11Z-eicosenoic acid, arachidonic acid, erucic acid, and 13Z,16Z-docosadienoic acid, further preferably IL-10, HGF, myristic acid, pentadecanoic acid, palmitic acid, margaric acid, isoheptadecanoic acid, stearic acid, oleic acid, linoleic acid, nonadecylic acid, erucic acid, 13Z,16Z-docosadienoic acid, and particularly preferably IL-10, HGF, palmitic acid, margaric acid, stearic acid, oleic acid, and 13Z,16Z-docosadienoic acid, and each of the exosomes in the present invention preferably contains them.

Palmitic acid in the present invention is a saturated fatty acid with a composition of 16:0 based on the number of carbon atoms as represented by C₁₆H₃₂O₂ and the number of double bonds. Stearic acid in the present invention is a saturated fatty acid with a composition of 18:0 based on the number of carbon atoms as represented by C₁₈H₃₆O₂ and the number of double bonds. Oleic acid in the present invention is a type of unsaturated fatty acids that is represented by C₁₈H₃₄O₂, and is a cis-monoene fatty acid.

The exosomes in the present invention may be ones contained in cells, or ones contained in a cell culture supernatant, or exosomes isolated from the inside of cells or from a cell culture supernatant. Examples of methods for isolating exosomes include an ultracentrifugation method, microfiltration, capture with an antibody, and use of a microfluid system. The isolated exosomes may include cells such as stromal cells (mesenchymal stem cells), and may include a medium such as a culture medium for stromal cells (mesenchymal stem cells).

The origin of the exosomes in the present invention is not particularly limited, and may be cells of a mammalian animal such as a human, a horse, cattle, a goat, a sheep, a pig, a dog, a cat, a rabbit, a mouse, and a rat, or a microorganism, or a plant such as rice. Exosomes can be collected from a culture supernatant obtained through culture of mesenchymal stem cells, or from a plant such as rice or microorganism that contains exosomes. Furthermore, the exosomes in the present invention are not limited to ones produced by an organism, and may be artificial endoplasmic reticula such as liposomes.

When the origin of the exosomes in the present invention is the mammalian animal, examples of origin cells include mesenchymal stem cells, liver-derived cells, fibroblasts, epithelial cells, myoblasts, pluripotent stem cells, and plant stem cells; mesenchymal stem cells and pluripotent stem cells are preferred among them; and mesenchymal stem cells are more preferred.

Hereinafter, a case in which the origin of the exosomes in the present invention is mesenchymal stem cells will be described.

When the cells are mesenchymal stem cells, the exosomes that the composition for treating sperm according to the present invention contains can be collected from a culture supernatant obtained through culture of mesenchymal stem cells. For example, mesenchymal stem cells are cultured in a culture vessel to a subconfluent or confluent status, the medium is exchanged with fresh one, the mesenchymal stem cells are further cultured for 1 to 5 days, and the culture supernatant is collected. The exosomes can be obtained by separating the culture supernatant for mesenchymal stem cells by means of ultracentrifugation, density gradient centrifugation, or any exosome separation kit or the like.

In addition to the exosomes, the composition for treating sperm according to the present invention may contain mesenchymal stem cells themselves that include the exosomes or have the ability to secrete them. When the composition for treating sperm contains mesenchymal stem cells that include the exosomes or have the ability to secrete them, it is even possible to interpret that containing the mesenchymal stem cells results in simultaneous satisfaction of the requirement of containing the exosomes. The exosomes that the composition for treating sperm according to the present invention contains are preferably exosomes isolated from a culture supernatant for mesenchymal stem cells or the like.

### (Mesenchymal stem cells)

In the present invention, the term "mesenchymal stem cells" refers to cells that have the ability to differentiate into one or more types of cells belonging to the mesenchymal lineages (such as osteocytes, cardiomyocytes, chondrocytes, tenocytes, adipocytes) and are capable of proliferating while maintaining that ability. The term "mesenchymal stem cells" used in the present invention refers to cells the same as stromal cells, and the two types of cells are not particularly distinguished from each other. The mesenchymal stem cells may be referred to as mesenchymal cells, simply. Examples of tissues containing mesenchymal stem cells include adipose tissue, umbilical cord, bone marrow, umbilical cord blood, endometrium, placenta, amnion, chorion, deciduum, dermis, skeletal muscle, periosteum, dental follicle, periodontium, dental pulp, and tooth germ. Examples of the mesenchymal stem cells in the present invention include those derived from adipose tissue, umbilical cord, bone marrow, umbilical cord blood, endometrium, placenta, amnion, chorion, deciduum, dermis, skeletal muscle, periosteum, dental follicle, periodontium, dental pulp, and tooth germ; adipose tissue-derived mesenchymal stem cells, umbilical cord-derived mesenchymal stem cells, and bone marrow-derived mesenchymal stem cells are preferred among them; and adipose tissue-derived mesenchymal stem cells and umbilical cord-derived mesenchymal stem cells are more preferred.

Examples of the species from which the mesenchymal stem cells in the present invention are derived include a human, a horse, cattle, a goat, a sheep, a pig, a dog, a cat, a rabbit, a mouse, and a rat. The mesenchymal stem cells in the present invention may be derived from a species the same as or different from that of a subject to be treated (test subject).

The mesenchymal stem cells may be cells, for example, provided by any of PromoCell, Lonza, Biological Industries, Veritas, R&D Systems, and Corning, or cells prepared with a method well known to those skilled in the art. The mesenchymal stem cells may be primary cells separated from tissue of a donor, or cells of an established cell line.

The medium used for culturing the mesenchymal stem cells in the present invention is not particularly limited as long as the medium allows the mesenchymal stem cells to be cultured while maintaining them in good condition and is preferably a medium that allows human mesenchymal stem cells to proliferate while maintaining the ability to differentiate, for example, into osteocytes, chondrocytes, and adipocytes.

The medium used in the present invention may be prepared by supplementing a basal medium with a serum and/or with one or more serum substitutes such as albumin, transferrin, fatty acid, insulin, sodium selenite, cholesterol, a collagen precursor, a trace element, 2-mercaptoethanol, or 3'-thiolglycerol. In addition, if necessary, the medium may be further supplemented with a substance such as an amino acid such as glutamine, a sugar such as glucose, a metal salt such as sodium chloride or magnesium sulfate, a trace metal such as selenium, a lipid such as cholesterol or unsaturated fatty acid, a vitamin such as pantothenic acid, a protein such as albumin, insulin, transferrin, a growth factor, a proliferation factor, or a cytokine, a polysaccharide, a low-molecular-weight compound, an antibiotic, an antioxidant, a pyruvic acid, a buffer, or an inorganic salt.

Examples of the basal medium include IMDM medium, Medium 199 medium, Eagle's Minimum Essential Medium (EMEM) medium, αMEM medium, Dulbecco's modified Eagle's Medium (DMEM) medium, Ham's F12 medium, RPMI 1640 medium, Fischer 's medium, MCDB201 medium, and a mixed medium thereof.

From the viewpoint of use for treating sperm, the medium that is used for culturing the mesenchymal stem cells for use in the present invention is preferably a xeno-free medium, which is free of a xeno-derived component such as a serum. Examples of such a medium include a medium provided as a pre-prepared medium for mesenchymal stem cells (stromal cells) such as Mesenchymal Stem Cell Growth Medium 2 (Ready-to-use, manufactured by PromoCell), Mesenchymal Stem Cell Growth Medium XF (Ready-to-use, manufactured by PromoCell), MSCGM BulletKittm, MSCGMtm Mesenchymal Stem Cell Growth Medium BulletKittm (manufactured by Lonza), a xeno-free medium for human mesenchymal stem cells (MSC NutriStem (registered trademark) XF, manufactured by Biological Industries), MesenCult-ACF Plus (manufactured by Veritas), StemXVivotm Serum-Free Human MSC Expansion Media (manufactured by R&D Systems, Corning), a serum-free medium for adipose-derived stem cells (KBM ADSC-4, manufactured by Kohjin Bio), and a serum-free medium for mesenchymal stem cells (R: STEM Medium for hMSC High Growth, manufactured by Rohto).

Examples of the serum described above include, but are not limited to, human serum, fetal bovine serum (FBS), bovine serum, calf serum, goat serum, horse serum, pig serum, sheep serum, rabbit serum, and rat serum. When a serum is used, a basal medium may be supplemented with 5 v/v% to 15 v/v%, preferably 10 v/v%, thereof.

### (Preparation of mesenchymal stem cell culture supernatant)

A supernatant for mesenchymal stem cells that is obtained with a method shown below can be used as the mesenchymal stem cell culture supernatant in the present invention. Alternatively, for example, a product obtained by removing unnecessary components from the supernatant by means of dialysis, ultrafiltration, or the like, a fraction obtained by fractionating the supernatant with a column or the like, a fraction selected with an antibody or the like against specific molecules, or a fraction obtained through a centrifugal operation may be used as the mesenchymal stem cell culture supernatant in the present invention.

A medium of the same type as the medium for culturing the mesenchymal stem cells can be used as the medium that is used in obtaining the culture supernatant. The method for obtaining the culture supernatant is not particularly limited as long as the method is suitable for culture of the respective mesenchymal stem cells, and an example is a method in which mesenchymal stem cells are cultured at a temperature of 20°C to 37°C under a 2% to 7% CO₂ environment and a 5% to 21% O₂ environment, preferably at room temperature to 37°C under a 5% CO₂ environment, and the culture supernatant is obtained.

The mesenchymal stem cell culture supernatant according to the present invention needs contact between the mesenchymal stem cells and the medium, and even a washing solution resulting from washing the mesenchymal stem cells with the medium can be used for the culture supernatant in the present invention. The time of contact between the mesenchymal stem cells and the medium is, for example, 14 days or less, preferably 10 days or less, further preferably 7 days or less, and more preferably 5 days or less. Specifically, and preferably, for example, the mesenchymal stem cells are made to a subconfluent or confluent status in a culture vessel, the medium is exchanged with fresh one, the mesenchymal stem cells are then further cultured for 1 to 5 days, and the culture supernatant is collected. The culture to obtain the culture supernatant may be plate culture with adhesion to a flask, or suspension/stirring culture with adhesion to microbeads or the like.

### (Isolation of exosomes)

When exosomes are isolated, the exosomes can be obtained by using a conventionally known method such as ultracentrifugation, density gradient centrifugation, affinity purification, size exclusion chromatography, or a method of separation with any exome separation kit or the like for the culture supernatant for the mesenchymal stem cells that has been collected with the method described above. Ultracentrifugation, affinity purification, and size exclusion chromatography are preferred as a method for separating exosomes.

The amount of the exosomes that the composition for treating sperm according to the present invention contains is 0.001 pg/mL or more or 100 ug/mL, preferably 0.005 pg/mL or more and 100 ug/mL or less, more preferably 0.01 pg/mL or more and 10 ug/mL or less, and particularly preferably 0.1 pg/mL or more and 100 pg/mL or less, as the concentration of the exosomes in the composition for treating sperm. By setting the amount of the exosomes that the composition for treating sperm according to the present invention contains within the above numerical range, the composition for treating sperm exerts excellent effects of increasing the motility of sperm and suppressing the acrosome reaction of sperm while suppressing damage to the sperm.

The composition for treating sperm according to the present invention may contain a further component as long as the further component does not impair the effect of the present invention, in addition to the exosomes. Examples of the further component include a protective agent such as dimethylsulfoxide (DMSO) or albumin, an antibiotic, a sugar, an amino acid, a vitamin, a carrier, an excipient, a disintegrant, a buffer, an emulsifier, a stabilizer, a preservative, an antiseptic, and physiological saline. In addition, a buffer solution that is commercially available as a buffer solution (washing solution) for an ovum or sperm or the like may be used. Examples of such a buffer solution include HTF Medium (manufactured by FUJIFILM Wako Pure Chemical Corporation) containing NaCl, KCl, KH₂PO₄, MgSO₄·7H₂O, CaCl₂·2H₂O, NaHCO₃, Glucose, Na-pyruvate, Na-lactate, Phenol Red, Hepes, Gentamicin Sulfate salt, and the like.

The species of sperm that are the target of the composition for treating sperm according to the present invention may be any mammalian animal, and examples thereof include a human, a horse, cattle, a goat, a sheep, a pig, a dog, a cat, a rabbit, a mouse, a rat, and a rare animal.

The composition for treating sperm according to the present invention is used as a sperm conditioning solution, a sperm dilution solution, a sperm preservation solution, an artificial insemination solution, an in vitro fertilization solution, a solution for improving sperm motility, a solution for retaining sperm fertility, or the like.

The sperm conditioning solution is a solution for washing sperm and even suspending sperm to recover sperm that show good motility, when providing sperm for the purpose of artificial insemination, in vitro fertilization, or the like. The sperm dilution solution is a solution used to dilute sperm to an appropriate concentration and inject the same into the uterus when carrying out artificial insemination. The sperm preservation solution is a solution that can be used for chilled preservation or frozen preservation of sperm. The artificial insemination solution is a solution used to increase the fertilization rate in artificial insemination. The in vitro fertilization solution is a solution used to increase the fertilization rate in in vitro fertilization. The solution for improving sperm motility is a solution used to improve the motility of sperm. The solution for retaining sperm fertility is a solution used to retain the fertility of sperm.

The composition for treating sperm according to the present invention can be prepared by a conventional method after mixing a further necessary component with the exosomes.

### [Agent for improving sperm motility and agent for retaining sperm fertility]

The agent for improving sperm motility and the agent for retaining sperm fertility according to the present invention are characterized by including exosomes. The agent for improving sperm motility and the agent for retaining sperm fertility according to the present invention can provide sperm having increased sperm motility and suppressed acrosome reaction, by including exosomes. Sperm treated with the agent for improving sperm motility or the agent for retaining sperm fertility according to the present invention can be expected to increase fertilization efficiency and conception rate in artificial insemination or in vitro fertilization, promote early development, or the like.

The agent for improving sperm motility and the agent for retaining sperm fertility according to the present invention are substantially the same as the composition for treating sperm described above, and thus for a specific description thereof, the description in the section Composition for treating sperm can be applied as it is by replacing the composition for treating sperm with the agent for improving sperm motility or the agent for retaining sperm fertility.

In a method for treatment with the agent for improving sperm motility and the agent for retaining sperm fertility according to the present invention, a state in which the agent for improving sperm motility or the agent for retaining sperm fertility is in contact with sperm is formed in vivo or in vitro. In vivo, it is considered to administer the agent for improving sperm motility or the agent for retaining sperm fertility to the seminal vesicle, which is an organ for storing sperm. As long as a state in which the agent for improving sperm motility or the agent for retaining sperm fertility according to the present invention is in contact with sperm is formed after administration, the administration site and the administration method are not particularly limited. On the other hand, in order to in vitro form a state in which the agent for improving sperm motility or the agent for retaining sperm fertility according to the present invention is in contact with sperm, for example, previously provided sperm and the agent for improving sperm motility or the agent for retaining sperm fertility according to the present invention are placed and mixed in an appropriate container in vitro and treated for a certain period of time. The treatment temperature is 4°C to 40°C, preferably 15°C to 40°C, more preferably 25°C to 39°C, and further preferably 36°C to 38°C, which is about the same as body temperature. The treatment time is 10 minutes to 48 hours, preferably 20 minutes to 24 hours, more preferably 30 minutes to 4 hours, and further preferably 1 hour to 2 hours.

Sperm treated with the agent for improving sperm motility or the agent for retaining sperm fertility according to the present invention can be used for artificial insemination or in vitro fertilization (increased success rate or efficiency), breeding of livestock (increased success rate or efficiency of artificial insemination, breeding or maintenance of species (for example, maintenance of endangered species, or maintenance or crossbreeding of a pet strain), treatment or improvement of various diseases associated with sperm disorder or caused mainly or secondarily by sperm disorder (for example, varicocele, male infertility, cryptorchism, X-ray irradiation, or sperm disorder after malignant tumor surgery or chemotherapy), preservation of sperm, improvement of the motility, retention of the fertility, or preservation of immature sperm, and the like.

When artificial insemination is carried out by using sperm treated with the agent for improving sperm motility or the agent for retaining sperm fertility according to the present invention (also including artificial insemination for the purpose of breeding of livestock, or breeding or maintenance of species), a state in which sperm having improved motility and/or retained fertility and an ovum (egg cell, egg) coexist is formed in vivo or in vitro. In order to form the coexistence state in vivo, sperm having improved motility and/or sperm having retained fertility are administered to (implanted into) a site in a living organism where an ovum is present. For example, in the case of a mammalian animal, sperm treated with the present agent are injected into the uterus. On the other hand, if the coexistence state is formed in vitro, an ovum provided, for example, by isolation from a living organism or distribution from a cell bank or the like and sperm treated with the present agent may be placed in the same container and incubated, or sperm having improved motility and/or sperm having retained fertility may be inserted into the ovum by using a micromanipulator or the like. A conventional method can be followed except for using the condition characteristic of the present invention, that is, using sperm treated with the agent for improving sperm motility or the agent for retaining sperm fertility according to the present invention.

### [Method for improving sperm motility and method for retaining sperm fertility]

The present invention also includes a method for improving sperm motility and a method for retaining sperm fertility, characterized by culturing sperm in a solution including exosomes. In the method for improving sperm motility and the method for retaining sperm fertility according to the present invention, by culturing sperm in a solution including exosomes, the motility of the sperm can be improved and/or increased or the fertility of the sperm can be retained, while maintaining the acrosome. Sperm having motility increased and acrosome reaction suppressed by the method for improving sperm motility or the method for retaining sperm fertility according to the present invention can be expected to, for example, increase fertilization efficiency or conception rate in artificial insemination or in vitro fertilization, or promote early development.

The method for improving sperm motility and the method for retaining sperm fertility according to the present invention are methods for treating sperm with the agent for improving sperm motility or the agent for retaining sperm fertility according to the present invention described above, and thus for a specific description thereof, the section Agent for improving sperm motility and agent for retaining sperm fertility can be referred to.

### EXAMPLES

Hereinafter, the present invention will be described in detail with reference to Examples and Test Examples, but the present invention is not limited by these Examples and the like.

### [Example 1: Preparation of exosomes 1]

Umbilical cord mesenchymal stem cells (C-12971 Human Mesenchymal Stem Cells from Umbilical Cord Matrix (hMSC-UC), manufactured by PromoCell) were cultured in RIM medium (serum-free medium for mesenchymal stem cells, containing EGF, bFGF, albumin, transferrin, and insulin, manufactured by Rohto) for 3 days, and a culture supernatant was obtained (hereinafter referred to as "3D RIM culture supernatant"). Thereafter, medium exchange was performed, culture was further performed with RIM medium for 1 day, and a culture supernatant was obtained (hereinafter referred to as "1D RIM culture supernatant"). From the 1D RIM culture supernatant and the 3D RIM culture supernatant, exosomes were obtained by using an ExoTrap Exosome Isolation Spin Column (hereinafter referred to as "1D exosomes" and "3D exosomes").

### [Example 2: Preparation of exosomes 2]

Adipose-derived stem cells (PT-5006 HADSC - Human Adipose-Derived Stem Cells, manufactured by LONZA) were cultured in RIM medium (serum-free medium for mesenchymal stem cells, containing EGF, bFGF, albumin, transferrin, and insulin, manufactured by Rohto) for 3 days, and a culture supernatant was obtained (hereinafter referred to as "ADSC culture supernatant"). Exosomes were obtained by using an ExoTrap Exosome Isolation Spin Column (hereinafter referred to as "ADSC exosomes").

### [Example 3: Lipidome analysis]

In the same manner as in Example 1, umbilical cord mesenchymal stem cells (C-12971 Human Mesenchymal Stem Cells from Umbilical Cord Matrix (hMSC-UC), manufactured by PromoCell) were cultured in RIM medium (serum-free medium for mesenchymal stem cells, containing EGF, bFGF, albumin, transferrin, and insulin, manufactured by Rohto) for 1 day or 3 days, and culture supernatants were obtained (hereinafter referred to as "1D RIM culture supernatant" and "2D RIM culture supernatant", respectively). From the 1D RIM culture supernatant and the 3D RIM culture supernatant, exosomes were obtained by using an ExoTrap Exosome Isolation Spin Column (hereinafter referred to as "1D exosomes" and "3D exosomes"). Fatty acids contained in these exosomes were quantified by GC-MS. The results are shown in Table 1.

**[Table 1]**

| | 1D exosomes | 3D exosomes |
|---|---|---|
| Caproic acid | 1.29 | 0.48 |
| Palmitic acid | 6.15 | 4.08 |
| Stearic acid | 4.13 | 1.84 |

### [Example 4: Mobility of sperm]

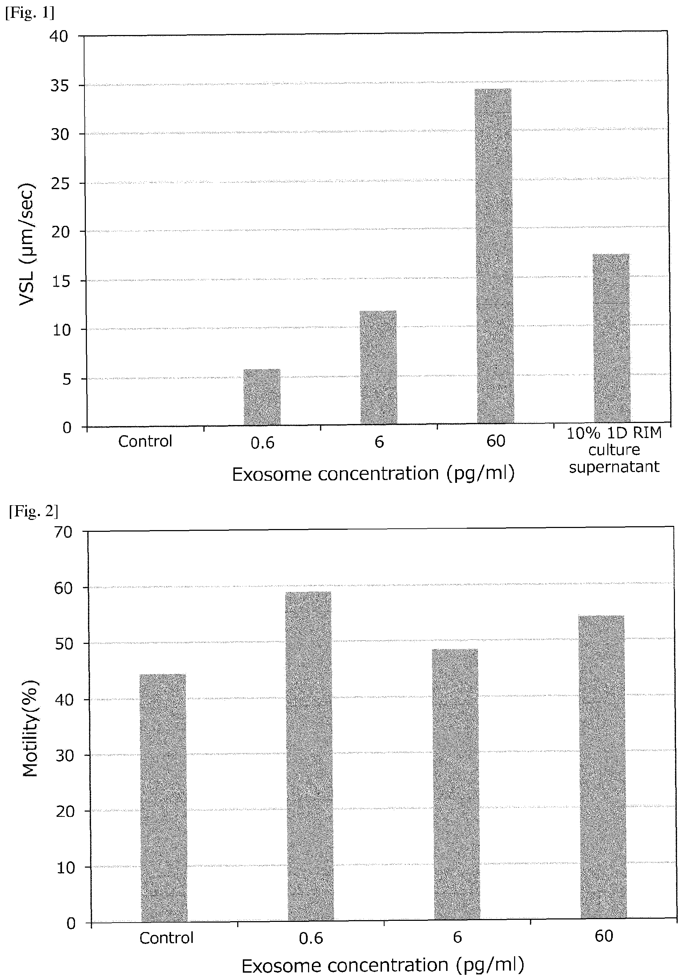

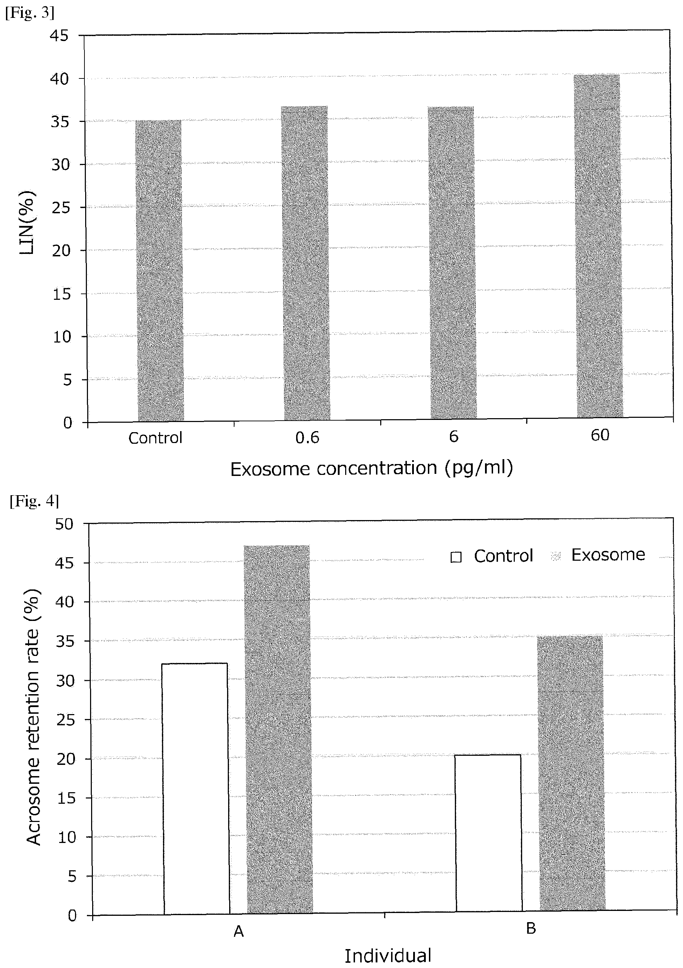

Mouse sperm (C57/BL6, 12 weeks old) were suspended in HTF Medium (manufactured by FUJIFILM Wako Pure Chemical Corporation) supplemented with 3D exosomes at different concentrations (3D exosome concentration: 0.6 pg/mL, 6.0 pg/mL, 60 pg/mL), HTF Medium supplemented with no exosome as negative control (control), and the ADSC culture supernatant (containing 60 pg/mL of exosomes) in a water bath at 37°C, and these were each used as a sample. Subsequently, the sample was cultured for 60 minutes at 37°C. Next, 3 µL of the sample was analyzed by using a sperm motility analyzer (Computer Assisted Sperm Analysis (CASA) system). The straight-line velocity (VSL), linearity (LIN), and motility (Motility) of all sperm observed were analyzed. With the median value for the control after culture for 60 minutes set to 100, the median value of VSL for the medium supplemented with 3D exosomes after culture for 60 minutes is shown (Fig. 1), and the mean values of Motility and LIN therefor are shown in Fig. 2 and Fig. 3.

As shown in Figs. 1 to 3, it was confirmed that the supplementation with exosomes increased the median value and increased the motility of the sperm. It was also confirmed that the isolated exosomes were superior in the effect of increasing the motility of sperm to the ADSC culture supernatant containing the same amount of exosomes.

### [Example 5: Suppression of acrosome reaction of sperm]

Sperm were collected from the epididymis of a male mouse (C57/BL6, 12 weeks old) and suspended in HTF Medium (manufactured by FUJIFILM Wako Pure Chemical Corporation) supplemented with 60 pg/mL of 3D exosomes and HTF Medium supplemented with no exosome as a negative control, and after culture for 60 minutes, the proportion of sperm retaining an acrosome was analyzed by counting positive sperm by visual observation. The proportions of the positive sperm retaining an acrosome are shown in Fig. 4.

As shown in Fig. 4, it was confirmed that the supplementation with exosomes suppressed the acrosome reaction of sperm.

### [Example 6: Motility of cattle sperm]

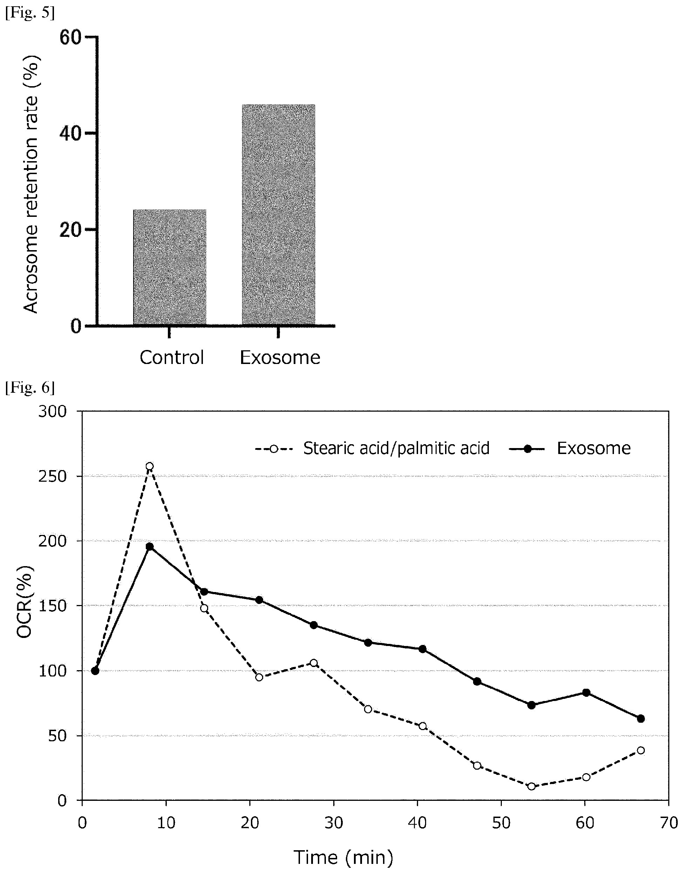

Frozen cattle semen (purchased from LIVESTOCK IMPROVEMENT ASSOCIATION OF JAPAN, INC.) was thawed by warming in a water bath at 37°C for 15 seconds, and the resultant was then washed twice with HTF Medium (manufactured by FUJIFILM Wako Pure Chemical Corporation). The sperm were suspended in HTF Medium (manufactured by FUJIFILM Wako Pure Chemical Corporation) supplemented with 60 pg/mL of 3D exosomes and HTF Medium supplemented with no exosome as a negative control, and after culture for 60 minutes, the proportion of sperm retaining an acrosome was analyzed by counting positive sperm by visual observation. The proportions of the positive sperm retaining an acrosome are shown in Fig. 5.

As shown in Fig. 5, it was confirmed that the supplementation with exosomes also suppressed the acrosome reaction of cattle sperm.

### [Example 7: Aerobic respiration]

Sperm were collected from the epididymis of a male mouse (C57/BL6, 12 weeks old) and suspended in HTF Medium (manufactured by FUJIFILM Wako Pure Chemical Corporation), and tested by flux analyzer analysis to measure the oxygen consumption rate of cells in real time. Six minutes after the start of culture, 20 µL of HTF Medium containing 60 pg/mL of 3D exosomes or stearic acid (8 ng)/palmitic acid (36 ng) as saturated fatty acids was added, and the change in oxygen consumption rate of the sperm was then examined every 6 minutes for 60 minutes. With the initial measurement for the control set to 100%, the change in oxygen consumption rate over time are shown in Fig. 6.

As shown in Fig. 6, it was confirmed that the supplementation with exosomes persistently promoted the aerobic respiration of sperm.

### [Example 8: Sperm staining]

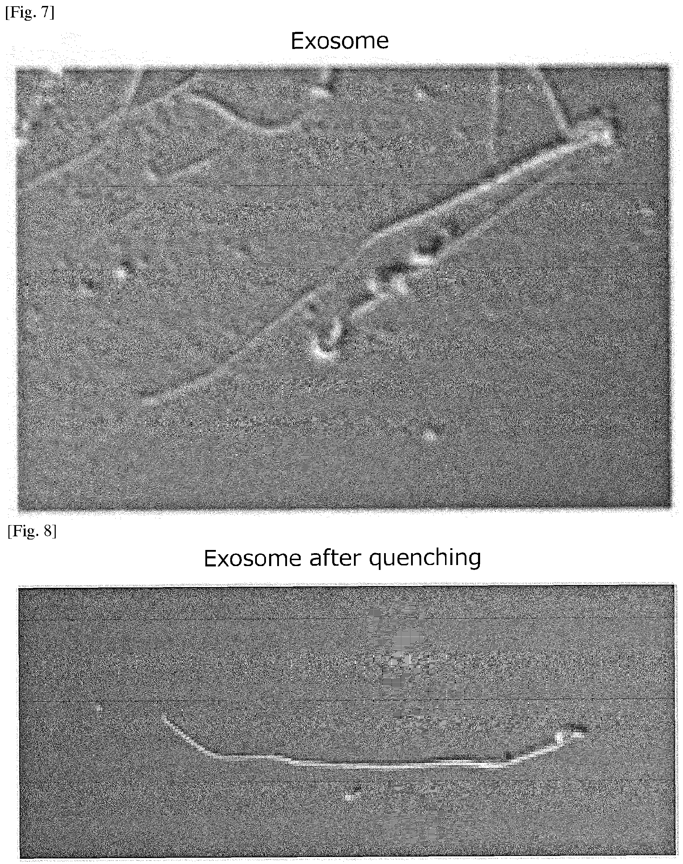

Sperm were collected from the epididymis of a male mouse (C57/BL6, 12 weeks old), suspended in HTF Medium (manufactured by FUJIFILM Wako Pure Chemical Corporation) supplemented with D3 exosomes fluorescence-labeled with an Exosparkler Exosome Membrane Labeling Kit (DOJINDO LABORATORIES), and cultured for 30 minutes at 37°C (Exosome). To select fluorescent labels incorporated in cells, quenching (culture with 0.4% trypan blue solution for 5 minutes) to deactivate extracellular fluorescent labels was performed (Exosome after quenching). The stained sperm were applied onto a microscope slide with a Cytospin, and observed with a fluorescence microscope. The fluorescence staining images are shown in Figs. 7 and 8.

As shown in Figs. 7 and 8, it was confirmed that stained exosomes were incorporated in sperm to cause membrane fusion.

### INDUSTRIAL APPLICABILITY

According to the present invention, it is possible to provide sperm with high motility and suppressed acrosome reaction. The sperm prepared by the present invention has higher motility and a suppressed acrosome reaction compared to sperm prepared by conventional methods, resulting in a significantly higher success rate of fertilization.

## Claims

1. A composition for treating sperm, comprising exosomes.

2. The composition for treating sperm according to claim 1, wherein the composition is a sperm conditioning solution, a sperm dilution solution, a sperm preservation solution, an artificial insemination solution, an in vitro fertilization solution, a solution for improving sperm motility, or a solution for retaining sperm fertility.

3. The composition for treating sperm according to claim 1, wherein the exosomes are derived from a mesenchymal stem cell culture supernatant.

4. The composition for treating sperm according to claim 1, wherein the exosomes are isolated exosomes.

5. An agent for improving sperm motility, comprising exosomes.

6. An agent for retaining sperm fertility, comprising exosomes.

7. A method for improving sperm motility, comprising culturing sperm in a solution comprising exosomes.

8. A method for retaining sperm fertility, comprising culturing sperm in a solution comprising exosomes.
